Europäisches Patentamt

European Patent Office

Office européen des brevets

(11) Publication number: **0 474 901 B1**

# EUROPEAN PATENT SPECIFICATION

(45) Date of publication of patent specification: **18.01.95** (51) Int. Cl.⁶: **C12N 5/04**

(21) Application number: **90117507.5**

(22) Date of filing: **11.09.90**

(54) **Method of culturing bupleurum protoplast.**

(43) Date of publication of application:
**18.03.92 Bulletin 92/12**

(45) Publication of the grant of the patent:
**18.01.95 Bulletin 95/03**

(84) Designated Contracting States:
**DE ES FR GB IT NL**

(56) References cited:
**US-A- 4 473 648**

**PATENT ABSTRACT OF JAPAN & JP-A-2
245180 (SHISEIDO CO LTD) 29 September
1990**

**PATENT ABSTRACTS OF JAPAN, vol. 13, no.
275 (C-610)(3623), 23 June 1989**

(73) Proprietor: **SHISEIDO COMPANY LIMITED
7-5-5, Ginza
Chuo-ku
Tokyo 104-10 (JP)**

(72) Inventor: **Gozu, Yoko, c/o Shiseido Laboratories
1050, Nippa-cho,
Kohoku-ku
Yokohama-shi,
Kanagawa (JP)**
Inventor: **Yokoyama, Mineyuki, c/o Shiseido
Laboratories
1050, Nippa-cho,
Kohoku-ku
Yokohama-shi,
Kanagawa (JP)**

(74) Representative: **Brandes, Jürgen, Dr. rer. nat.
Wuesthoff & Wuesthoff
Patent- und Rechtsanwälte
Schweigerstrasse 2
D-81541 München (DE)**

**Description**

TECHNICAL FIELD

This invention relates to a method of culturing Bupleurum protoplast. The method of this invention can be widely utilized for the breeding of Bupleurum and the production of saponin, etc.

BACKGROUND ART

Although techniques such as cell fusion and gene introduction are widely used in the breeding of plants by plant tissue culture techniques, to utilize these techniques, a protoplast culture system must be established in each plant species. Regarding the culturing of protoplasts, a report was made on tobacco, by Takebe, et al. (Planta, 99, 12-20, 1971), and the culturing of numerous plant species has been reported. Recently, even in the culturing of rice plants, which has been considered difficult, successful examples thereof have been reported (Bio. Technology, 4, 1085-1090, 1986; Plant Cell Reports, 5, 85-88, 1986; Plant Science, 47, 123-133, 1986). Nevertheless, their culturing methods and culturing conditions of different plant species are different, and culturing methods successfully used for other species cannot be always applied, and thus it is necessary to establish a protoplast culturing system for each plant species.

Although Bupleurum is an important medicinal plant which has widely been used as an galenical, specifically as an antiphlogistic, antipyretic or analgesic, a successful example of a protoplast culturing thereof has not been reported.

Therefore, when breeding an important medicinal plant Bupleurum by plant tissue culture techniques, the development of a protoplast culture system suitable for Bupleurum protoplast is desired. According to conventional methods, the present inventors isolated protoplasts derived from Bupleurum, purified and cultured them under various conditions, and investigated their culture conditions. As a result they found that, when the protoplasts are cultured in a plant tissue culturing medium containing a specified vitamin, organic acid or the like, they start to divide and form callus.

SUMMARY OF THE INVENTION

Thus, the object of this invention is to provide a method of culturing protoplasts derived from Bupleurum, which comprises using a plant tissue culturing medium containing a specified vitamin, organic acid or the like. In more detail, the culturing method of this invention is carried out by using a plant tissue culturing medium containing, in addition to a conventional plant tissue culturing basal medium, nicotinic acid, pyridoxine hydrochloride, thiamine hydrochloride, calcium pantothenate, p-aminobenzoic acid, choline chloride, ascorbic acid, vitamin A, vitamin $D_3$, vitamin $B_{12}$, sodium pyruvate, citric acid, sodium malate, fumaric acid and casein hydrolyzate.

DESCRIPTION OF THE PREFERRED EMBODIMENT

Protoplasts used in this invention can be prepared by thoroughly immersing cells of Bupleurum falcatum L. for 2 to 3 hours, according to a conventional method, in an enzyme solution which contains cellulase and pectinase and having an osmotic pressure thereof adjusted with mannitol, and then washing the resulting protoplasts with a suitable washing liquid. It is preferable to use cells derived from a callus induced from any of a leaf, a stem, and a root of Bupleurum falcatum L. The protoplasts prepared in the above manner can be advantageously cultured in the above medium of the invention. Although the content of the above medium components, i.e., each vitamin and each organic acid in the medium, is not limited because the optimum amount thereof differs according to respective combinations, the medium contains all of the above respective components; for example, contains 0.1 to 5.0 $\mu$g/ml, preferably 0.5 to 1.0 $\mu$g/ml nicotinic acid, 0.1 to 5.0 $\mu$g/ml, preferably 0.5 to 1.0 $\mu$g/ml pyridoxine hydrochloride, 0.1 to 20.0 $\mu$g/ml, preferably 1.0 to 10.0 $\mu$g/ml thiamine hydrochloride, 0.1 to 5.0 $\mu$g/ml, preferably 0.5 to 1.0 $\mu$g/ml calcium D-pantothenate, 0.01 to 2.0 $\mu$g/ml, preferably 0.01 to 0.5 $\mu$g/ml p-aminobenzoic acid, 0.1 to 5.0 $\mu$g/ml, preferably 0.5 to 1.0 $\mu$g/ml choline chloride, 0.1 to 10.0 $\mu$g/ml, preferably 0.1 to 5.0 $\mu$g/ml ascorbic acid, 0.01 to 1.0 $\mu$g/ml, preferably 0.01 to 0.1 $\mu$g/ml vitamin A, 0.01 to 1.0 $\mu$g/ml, preferably 0.01 to 0.1 $\mu$g/ml vitamin $D_3$, 0.01 to 1.0 $\mu$g/ml, preferably 0.01 to 0.1 $\mu$g/ml vitamin $B_{12}$, 1.0 to 40.0 $\mu$g/ml, preferably 10.0 to 30.0 $\mu$g/ml sodium pyruvate, 10.0 to 80.0 $\mu$g/ml, preferably 20.0 to 50.0 $\mu$g/ml citric acid, 10.0 to 80.0 $\mu$g/ml, preferably 20.0 to 50.0 $\mu$g/ml sodium malate, 10.0 to 80.0 $\mu$g/ml, preferably 20.0 to 50.0 $\mu$g/ml fumaric acid, and/or 50.0 to 500.0 $\mu$g/ml, preferably 100.0 to 300.0 $\mu$g/ml Casamino acid. The protoplast culturing medium used in the method of this invention preferably contains, besides the above various components, i.e., vitamins, organic acids, etc., 0.5 to 5 $\mu$g/ml, preferably 1.0 to 2.0 $\mu$g/ml auxins and 1 to 10%, preferably 3 to 6% carbon source having an osmotic pressure adjusted to 0.3 to 0.7 M, preferably 0.4 to 0.5 M, with mannitol. As the auxins, there can be added 2,4-dichlorophenoxyacetic acid (hereinafter abbreviated as 2,4-D), $\alpha$-naphthaleneacetic acid (hereinafter abbreviated as NAA), indole-3-acetic acid, indole-3-butyric acid, 2,4,5-trichlorophenoxyacetic acid, etc., and as the carbon source, glucose or sucrose can be used. It is preferably an insufficient osmotic pressure is used, regardless of the use of glucose or sucrose with a metabolically inactive saccharide such as mannitol or sorbitol. As the basal medium of the plant tissue culturing medium used in the invention, there can be mentioned Murashige & Skoog medium, Linsmaicr & Skoog medium (hereinafter abbreviated as LS medium), V47 medium, V-KM medium, B5 medium, KM8p medium or WB medium, and further be, a medium consisting solely of the inorganic salts of each of the above media can be used.

Protoplasts derived from Bupleurum falcatum L. have a strong flocculant property, and therefore, if directly dispersed into a liquid medium, rapidly flocculate. This is not preferable from the viewpoint of the subsequent division thereof. Thus, preferably a method is used wherein the protoplasts are embedded in the above medium, to which a coagulant known per se was added. The protoplasts embedded in the medium can be standing cultured under darkness or under illumination at about 20 to 30 °C for about 30 to 40 days, to form colonies, and the thus-obtained colonies can be implanted in an appropriate medium according to a conventional method, to form a callus. The resulting callus can be habituated and bred according to conventional method, to obtain Bupleurum falcatum L. plants.

Example

This invention is now specifically described below by way of examples, which in no way limit the present invention.

A liquid culture was carried out by suspending a callus induced from a leaf, stem on root of Bupleurum falcatum L., according to a conventional method, in an LS liquid medium to which 0.5 $\mu$g/ml 2.4-D had been added as a plant hormone. The liquid suspension culture cells were subcultured weakly, and those at the stage at which the growth thereof was flourishing were used as a material for an isolation of protoplasts thereof.

To the liquid suspension culture cells was added an enzyme solution containing cellulase and pectinase having an osmotic pressure which had been adjusted to 0.5 M with mannitol, and the mixture was gently shaken at 27 °C in a dark place. After about 2.5 hours of the enzyme treatment, the mixture was filtered through 82 $\mu$m nylon mesh to remove untreated cell lumps and cell residues. The resulting protoplasts were washed with a washing solution having the following composition:

| | |
|---|---|
| $KH_2PO_4$ | 27.2 mg/l |
| $KNO_3$ | 101 mg/l |
| $CaCl_2 \cdot 2H_2O$ | 1480 mg/l |
| $MgSO_4$ | 240 mg/l |
| KI | 0.16 mg/l |
| $CuSO_4 \cdot 5H_2O$ | 0.025 mg/l |
| Mannitol | 90 g/l |

Further, basal media 1 to 4 having the compositions shown in Table 1 were prepared, plant hormones and a carbon source were added to these basal media 1 to 4, respectively, as shown in Table 2, and then the osmotic pressures thereof were adjusted with mannitol to prepare plant tissue culturing media.

Protoplasts obtained after washing were embeded in each of the thus-obtained media, so that the concentration thereof became $2 \times 10^4$ to $5 \times 10^5$/ml (refer to Table 2), and the culturing thereof was started at 27 °C in a dark place. In this connection, 0.15% gelan gum was used as a coagulant. The protoplasts began a first division thereof 2 to 3 days after the start of the culture, and grew into colonies on the order of 0.2 to 0.5 mm, 1 to 2 months thereafter. The emergence frequency results of the colonies are shown in Table 2. When the colonies thus emerged were implanted in usual callus subculturing media (LS agar media to which 1 ppm 2.4-D and 0.1 ppm KT were added), they grew normally into a callus.

3

## Table 1

| Components of basal medium | Basal medium (present invention) | Basal medium (for comparison) | Basal medium (present invention) | Basal medium (for comparison) |
|---|---|---|---|---|
| $NH_4NO_3$ | 600 | 1650 | 1650 | 720 |
| $KNO_3$ | 1900 | 1900 | 1900 | 950 |
| $CaCl_2 \cdot 2H_2O$ | 600 | 440 | 440 | 166 |
| $MgSO_4 \cdot 7H_2O$ | 300 | 370 | 370 | 185 |
| $KH_2PO_4$ | 170 | 170 | 170 | 68 |
| KCl | 300 | - | - | - |
| $FeSO_4 \cdot 7H_2O$ | 28 | 28 | 28 | 28 |
| $Na_2EDTA$ | 37 | 37 | 37 | 37 |
| $H_3BO_3$ | 3 | 6.2 | 6.2 | 10 |
| $MnSO_4 \cdot H_2O$ | 10 | 22.3 | 22.3 | 25 |
| $ZnSO_4 \cdot 7H_2O$ | 2 | 8.6 | 8.6 | 10 |
| $NaMoO_4 \cdot 2H_2O$ | 0.25 | 0.25 | 0.25 | 0.25 |
| $CuSO_4 \cdot 5H_2O$ | 0.025 | 0.025 | 0.025 | 0.025 |
| $CoCl \cdot 6H_2O$ | 0.025 | 0.025 | 0.025 | - |
| KI | 0.75 | 0.83 | 0.83 | - |
| Inositol | 100 | 100 | 100 | 100 |
| Nicotinic acid | 1 | - | 1 | 5 |
| Pyridoxine HCl | 1 | - | 1 | 0.5 |
| Thiamine HCl | 10 | 0.4 | 10 | 0.5 |
| Calcium pantothenate | 1 | - | 1 | - |
| Folic acid | 0.4 | - | 0.4 | 0.5 |

## Table 1 (Continued)

| Components of basal medium | Basal medium (present invention) | Basal medium (for comparison) | Basal medium (present invention) | Basal medium (for comparison) |
|---|---|---|---|---|
| p-Aminobenzoic acid | 0.02 | - | 0.02 | - |
| Biotin | 0.01 | - | 0.01 | 0.05 |
| Choline chloride | 1 | - | 1 | - |
| Ascorbic acid | 2 | - | 2 | - |
| Vitamin A | 0.01 | - | 0.01 | - |
| Vitamin $D_3$ | 0.01 | - | 0.01 | - |
| Vitamin $B_{12}$ | 0.02 | - | 0.02 | - |
| Glycine | 2 | - | 2 | 2 |
| Sodium pyruvate | 20 | - | 20 | - |
| Citric acid | 40 | - | 40 | - |
| Sodium malate | 40 | - | 40 | - |
| Fumaric acid | 40 | - | 40 | - |
| Casein hydrolyzate | 250 | - | 250 | - |

## Table 2

| Basal medium | Plant hormone (ppm) | Carbon source (%) | Osmotic pressure (M) | Protoplast concentration (protoplasts /ml) | Colony emergence frequency (%) |
|---|---|---|---|---|---|
| 1 | NAA1.5+KT1.0 | Glu 3 | 0.5 | $4.9 \times 10^4$ | 0.34 |
|   | " | " | 0.6 | " | 0.09 |
|   | " | " | 0.7 | " | 0.002 |
|   | NAA1.5 | Glu 1 | 0.5 | $3 \times 10^4$ | 0.009 |
|   | " | 3 | " | " | 0.017 |
|   | " | 4 | " | " | 0.084 |
|   | " | 6 | " | " | 0.073 |
|   | NAA1.5 | Glu 4 | 0.3 | $6 \times 10^4$ | 0.075 |
|   | " | " | 0.35 | " | 0.035 |
|   | " | " | 0.4 | " | 0.043 |
|   | " | " | 0.5 | " | 0.016 |
| 2 | NAA1.5 | Glu 3 | 0.4 | $3 \times 10^5$ | - |
|   | " | " | 0.5 | " | - |
|   | " | Glu 9 | 0.4 | " | - |
|   | " | " | 0.5 | " | - |
| 3 | NAA1.5+KT1.0 | Glu 3 | 0.6 | $4.9 \times 10^5$ | 0.01 |
|   |   | " | 0.65 | " | 0.004 |
|   |   | " | 0.7 | " | 0.004 |
| 4 | NAA1.5+KT1.0 | Glu 3 | 0.5 | $2 \times 10^4$ | - |
|   |   | " | 0.6 | " | - |
|   |   | " | 0.7 | " | - |

Note: NAA ... α-Naphthaleneacetic acid
KT .... Kinetin
Glu ... Glucose

## Claims

1. A method of culturing protoplasts derived from Bupleurum, which comprises culturing the protoplasts in a plant tissue culturing medium containing nicotinic acid, pyridoxine hydrochloride, thiamine hydrochloride, calcium pantothenate, p-aminobenzoic acid, choline chloride, ascorbic acid, vitamin A, vitamin $D_3$, vitamin $B_{12}$, sodium pyruvate, citric acid, sodium malate, fumaric acid and casein hydrolyzate.

2. The method according to claim 1 wherein the protoplasts are cultured such that the protoplasts are embedded in a plant tissue culturing medium to which a coagulant is added.

**Patentansprüche**

1. Verfahren zur Kultivierung von Protoplasten, die sich von <u>Bupleurum</u> ableiten, bei dem man die Protoplasten in einem Pflanzengewebekulturmedium kultiviert, das Nikotinsäure, Pyridoxinhydrochlorid, Thiaminhydrochlorid, Calciumpantothenat, p-Aminobenzoesäure, Cholinchlorid, Ascorbinsäure, Vitamin A, Vitamin $D_3$, Vitamin $B_{12}$, Natriumpyruvat, Zitronensäure, Natriummalat, Fumarsäure und Kaseinhydrolysat enthält.

2. Verfahren nach Anspruch 1, bei dem die Protoplasten derart kultiviert werden, daS die Protoplasten in ein Pflanzengewebekulturmedium eingebettet werden, dem ein Koagulierungsmittel zugegeben wird.

**Revendications**

1. Procédé pour cultiver des protoplastes dérivés de <u>Bupleurum</u>, qui comprend la culture des protoplastes dans un milieu de culture de tissu végétal contenant de l'acide nicotinique, du chlorhydrate de pyridoxine, du chlorhydrate de thiamine, du pantothénate de calcium, de l'acide p-aminobenzoïque, du chlorure de choline, de l'acide ascorbique, de la vitamine A, de la vitamine $D_3$, de la vitamine $B_{12}$, du pyruvate de sodium, de l'acide citrique, du malate de sodium, de l'acide fumarique et un hydrolysat de caséine.

2. Procédé suivant la revendication 1, dans lequel les protoplastes sont cultivés tandis que les protoplastes sont noyés dans un milieu de culture de tissu végétal additionné d'un coagulant.